# EUROPEAN PATENT APPLICATION

(11) **EP 4 830 697 A1**
(43) Date of publication of application: **28.10.2026**
(21) Application number: 26171493.5
(22) Date of filing: 09.04.2026
(51) Int. Cl.: A61K 9/00, A61K 9/08, A61K 31/728, A61K 38/28, A61K 47/36

(54) **TOPIC OCULAR FORMULATION**

(30) Priority: 24.04.2025 IT 202500009189
(71) Applicant: Limoli, Paolo Giuseppe, 90133 Palermo (PA) (IT)
(72) Inventor: Limoli, Paolo Giuseppe, 90133 Palermo (PA) (IT)
(74) Representative: Long, Giorgio

(57) **Abstract**

The subject of this invention is a topical ocular formulation for the treatment of neurodegenerative diseases affecting the eye.

In particular, the present invention concerns a topical ocular formulation including insulin and hyaluronic acid or a salt thereof and said formulation for use in the treatment of a neurodegenerative eye disease.

## Description

### Field of the invention

The subject of this invention is a topical ocular formulation for the treatment of neurodegenerative diseases affecting the eye.

### State of the art

Eye diseases are diseases that affect one or more structures of the eye. Among them, there are serious diseases, such as neurodegenerative diseases of the retina.

These diseases are characterized by triggering for inflammatory, vascular or genetic reasons affecting the neuroretina (pigment epithelial cells, photoreceptors, ganglion cells and their nerve fibers) with impairment of the intrinsic metabolism of residual cells.

The residual neuroretina is influenced by the pathological conditions of the underlying disease and cellular suffering depresses cellular metabolism with consequent functional incapacity of the residual cell (silent cells) and inability to repair the oxidative and inflammatory processes triggered by the underlying disease.

Generally, there are no therapies for these diseases except to contain risk factors (e.g. hypertonicity, ischemia, inflammation) and complications (e.g. exudations).

There is therefore a need to provide a therapy that can improve eye conditions or at least stabilize the advancement of these diseases in a simple and minimally invasive way.

### Summary of the invention

The aforementioned need is addressed by the present invention, which proposes a topical ocular formulation containing insulin and hyaluronic acid.

The present invention therefore concerns a topical ocular formulation as outlined in the attached claims.

An object of the invention is in particular a topical ocular formulation including insulin and hyaluronic acid or a salt thereof.

A further object of the invention is the aforementioned formulation for use in the treatment of a neurodegenerative eye disease.

These and other objects will be described later in the description. The text of the claims shall be deemed to be included in the description for the purposes of assessing the sufficiency of the description.

Further characteristics and advantages of the invention will be shown in the description below of preferred examples of realization, given by way of example and not exhaustively.

### Detailed description of the invention

The present invention concerns a topical ocular formulation including insulin and hyaluronic acid or a salt thereof.

In preferred embodiments, insulin is recombinant human insulin. Used to treat diabetes, recombinant human insulin is the first biologic drug to be approved in 1982.

It has been seen that the insulin molecule, by binding to a specific membrane receptor,
- facilitates the passage of glucose from the blood to the cells, which use it as an energy source (lowering blood sugar),
- facilitates the passage of amino acids from the blood to the cells, favoring the use of cytokines that spread via paracrine from the mesenchymal secretome,
- facilitates the passage of potassium inside the cells, contributing to the maintenance of the membrane gradient and therefore favoring the excitability of the nerve cell,
- stimulates cell proliferation, maintaining cell trophism,
- it is able to activate signaling pathways such as oxidative phosphorylation capable of converting glucose into simpler molecules and producing energy in the form of adenosine triphosphate.

Hyaluronic acid, normally used as sodium hyaluronate, is a polymer consisting of a particular molecular structure which, thanks to its hygroscopic properties, helps lubricate, hydrate and protect the ocular surface, stabilizing the aqueous layer of the tear film. In addition, it has been noted that the muco-adhesive properties and lubricating action of hyaluronic acid help the physiological repair processes of the corneal epithelium.

The present invention is based on the evidence that the use of pure insulin can impact the patient's compliance in taking it, producing burning and reactive tearing, with consequent dispersion of the active ingredient.

The integration of the active ingredient in a formulation with hyaluronic acid, which is more physiological and biocompatible, creates a synergism useful in promoting its enhancement. In this way, it is possible to reduce the overall amount of insulin as the hyaluronic acid solution allows the active ingredient to remain on the ocular surface for a longer time, favoring its absorption.

In addition, the ocular surface is respected, restoring homeostasis with a significant relief of symptoms during instillation.

In fact, studies show that the incorporation of polymers (such as hyaluronic acid) into delivery vehicles is a useful approach to prolong the residence time of insulin in the precorneal tear film and to improve penetration into biological membranes by enhancing their effect. This synergism favors the goal of formulating new eye drops.

The applicant noted that the increased mucoadhesiveness of the combination with hyaluronic acid contributes to the synergistic enhancement of the composition.

Hyaluronic acid or its salt is preferably high molecular weight hyaluronic acid, i.e. having a molecular weight greater than 2 million Daltons.

The formulation of the invention can be in the form of eye drops, cream, ointment or solution for intraocular infiltration. In a particularly preferred embodiment, the formulation is in the form of eye drops.

In certain embodiments, the weight ratio between insulin and hyaluronic acid is between 37:1 and 33:1.

In preferred embodiments, the formulation of the invention is eye drops comprising or consisting of 75-85%, preferably 79-81%, by volume of insulin solution at a concentration of 100 U/ml and 14-25%, preferably 14-21%, by volume of a solution of hyaluronic acid at a concentration of 0.4% w/v.

In these solutions, the solvent is preferably sterile water.

Insulin is preferably insulin lispro, i.e. insulin originating from recombinant DNA produced in *E. coli.* The property of insulin lispro to make glucose molecules penetrate cells and therefore lower blood sugar faster than human insulin is exploited to increase cellular metabolism.

For example, the product called HUMALOG can be used, containing, in addition to insulin lispro, the following excipients: metacresol (3.15 mg/ml), glycerol, dibasic sodium phosphate, zinc oxide, water for injections, hydrochloric acid and sodium hydroxide (to adjust the pH).

In certain embodiments, the formulation may also contain vitamin B5 in a percentage by weight/volume between 3 and 7%, preferably about 5%. Vitamin B5, or *pantothenic acid,* is one of the so-called water-soluble vitamins. Characterized by antioxidant activity, it acts as a free radical scavenger, ensuring the maintenance of the physical characteristics of the formulation. Due to its hygroscopic nature, it prevents epithelial dryness, moisturizes the tear film and maintains the integrity of the tear film and ocular surface.

In certain embodiments, the formulation may also contain EDTA in concentrations between 0.003 and 0.007%, preferably about 0.005% by weight/volume. EDTA (i.e., *ethylenediaminetetraacetic acid)* is a chelating substance, capable of forming stable and soluble complexes with calcium and, for this reason, can be used topically in the treatment of corneal opacifications and calcium deposits in the eye. This substance binds metal ions forming stable water-soluble complexes and making them unavailable to catalyze both oxidation reactions and promotion of microbial growth. It can be used in synergy with antibacterials and antioxidants in the preservation of drugs containing metal ions.

In certain embodiments, the formulation may also contain sodium metabisulfite in concentrations between 0.20 and 0.30%, preferably about 0.25% by weight/volume. Sodium metabisulphite has as its main characteristic the release of sulfur dioxide which has oxidizing and antibacterial properties, inhibiting the growth of bacteria or molds.

It acts as a stabilizer of the solution. Compared to other stabilizers such as Vitamin E-TPGS, which is fat-soluble, sodium metabisulfite is water-soluble and can go into aqueous solution.

Formulations according to the invention can be prepared according to conventional methods, such as those described in Remington's Pharmaceutical Sciences Handbook, Mack Pub. Co., N.Y., USA, 17th edition, 1985 or in Remington, The Science and Practice of Pharmacy, Edited by Allen, Loyd V. , Jr, 22nd edition, 2012 or in subsequent editions.

### EXPERIMENTAL PART

The effect of the formulation according to the invention in various degenerative diseases was evaluated, in particular: GON (glaucomatous optic neuropathy), MMA (myopic macular atrophy or atrophic degenerative myopia), AMD (age related macular degeneration), RP (retinitis pigmentosa or retinitis pigmentosa), OMD (occult macular dystrophy), RD (diabetic retinopathy), optic atrophy, cone dystrophy.

Infiltration was performed in a large sample of eyes that had previously performed a graft of subscleral mesenchymal cells (adipose peduncle, stem cells and concentrated platelets) in order to contain the progression of damage or enhance residual cell viability. Therefore, it is a single infiltration at the pedicle level made on previously implanted pedicles, performed on the occasion of a periodic treatment, usually annual or six-monthly, of mesenchymal secretome (from concentrated platelets).

Eye sensitivity, measured in decibels (dB), indicates the retina's ability to perceive light. The higher the sensitivity, the greater the ability to see. Visual field examination, for example, measures retinal sensitivity in decibels to assess retinal function in different areas. Sensitivity is assessed with microperimetry.

Infiltration treatment resulted in significant increases in ocular sensitivity, greater than 5%, with a gap between treated and untreated eye of more than 8%.

Treatment with eye drops (1 drop after breakfast, instilled in the conjunctival sac) resulted on average, after one year of administration, in a smaller increase, i.e. 2.95%, in eye sensitivity, but demonstrated the ability to avoid the progression of damage one year after the start of treatment. This effect of stabilizing pathology is a very important aspect of the present invention.

If the effect on specific pathologies is also observed, it can be seen that in retinitis pigmentosa (12 eyes treated with eye drops) the sensitivity went from 7.72 to 8.43 dB with an increase of 9.23%; in optic atrophy (5 eyes) from 18.67 to 20.22 dB with an increase of 8.35%; in Cone distrophy (4 eyes) from 21.40 to 21.76 dB with an increase of 1.67%.

It is clear that the effect on metabolic activity could be different depending on the pathology considered, indicating how, for example, retinitis pigmentosa and optic atrophy are more sensitive to treatment with the formulation of the invention.

Therefore, an additional object of the present invention is an ocular topical formulation as defined above for use in the treatment of neurodegenerative diseases of the eye, in particular glaucomatous optichopathy, atrophic degenerative myopia, age-related macular degeneration, retinitis pigmentosa, occult macular dystrophy, diabetic retinopathy, optic atrophy and cone dystrophy, more particularly retinitis pigmentosa and optic atrophy.

## Claims

1. A topical ocular formulation including insulin and hyaluronic acid or a salt thereof.

2. The formulation according to claim 1, where insulin is human recombinant insulin.

3. the formulation according to claim 2, where insulin is insulin lispro originating from recombinant DNA produced in *E. coli.*

4. The formulation according to any of claims 1 to 3, where the hyaluronic acid or its salt is high molecular weight hyaluronic acid having a molecular weight greater than 2 million Daltons.

5. The formulation according to any of claims 1 to 4, where the formulation is in the form of eye drops, cream, ointment, or intraocular infiltration solution.

6. The formulation according to any of claims 1 to 4, where the formulation is eye drops comprising or consisting of 75-85%, preferably 79-81%, by volume of aqueous solution of insulin at a concentration of 100 U/ml and 14-25%, preferably 14-21%, by volume of an aqueous solution of hyaluronic acid at a concentration of 0.4% w/v.

7. The formulation according to any of claims 1 to 6, where the weight ratio of insulin to hyaluronic acid is between 37:1 and 33:1.

8. The formulation according to any of claims 6 to 7, in which the insulin solution includes the following excipients: metacresol (3.15 mg/ml), glycerol, dibasic sodium phosphate, zinc oxide, water for injections, hydrochloric acid and sodium hydroxide.

9. The formulation according to any of Claims 1 to 8, comprising one or more of the following components:
- vitamin B5 in percentage by weight/volume between 3 and 7%, preferably about 5%;
- EDTA in a concentration between 0.003 and 0.007%, preferably about 0.005% by weight/volume;
- sodium metabisulphite in concentrations between 0.20 and 0.30%, preferably about 0.25% by weight/volume.

10. A topical ocular formulation according to any of claims 1 to 9 for use in the treatment of neurodegenerative diseases of the eye.

11. The formulation for use according to claim 10, in which said pathologies are chosen from glaucomatous optichopathy, atrophic degenerative myopia, age-related macular degeneration, retinitis pigmentosa, occult macular dystrophy, diabetic retinopathy, optic atrophy, and cone dystrophy.

12. The formulation for use according to claim 11, where said pathologies are retinitis pigmentosa or optic atrophy.
